Europäisches Patentamt

European Patent Office

Office européen des brevets

(19)

(11) EP 1 580 575 A2

(12) EUROPEAN PATENT APPLICATION

(43) Date of publication:
28.09.2005 Bulletin 2005/39

(51) Int Cl.$^7$: G01T 1/164

(21) Application number: 05251405.6

(22) Date of filing: 08.03.2005

(84) Designated Contracting States:
AT BE BG CH CY CZ DE DK EE ES FI FR GB GR
HU IE IS IT LI LT LU MC NL PL PT RO SE SI SK TR
Designated Extension States:
AL BA HR LV MK YU

(30) Priority: 15.03.2004 JP 2004071978

(71) Applicant: GE Medical Systems Global
Technology Company LLC
Wisconsin 53188-1696 (US)

(72) Inventors:
• Nukui, Masatake
  Hino-shi Tokyo 191-8503 (JP)
• Gohno, Makoto
  Hino-shi Tokyo 191-8503 (JP)
• Imai, Yasuhiro
  Hino-shi Tokyo 191-8503 (JP)

(74) Representative: Goode, Ian Roy
London Patent Operation,
General Electric International Inc.,
15 John Adam Street
London WC2N 6LU (GB)

(54) Cross-talk correction method and X-ray CT apparatus

(57) For the purpose of providing a cross-talk correction method for easily removing leakage of fluorescent light occurring between scintillators adjacent in a slice direction, first function fitting means (51) in a channel direction and second function fitting means (53) in a slice direction determine an ideal projection length, In $(D_n)$, from slope phantom projection information (41), Equation (1) is then used to determine leakage coefficients $\varepsilon_+$ and $\varepsilon_-$, and subsequently, cross-talk removing means (82) determines intensity information $D_n$ from detected information $S_n$ making up subject projection information (42) on a subject using Equation (2) and the leakage coefficients $\varepsilon_+$ and $\varepsilon_-$; thus, the leakage component for scintillators adjacent in the slice direction contained in the subject projection information (42) is removed by a simple method to eliminate artifacts due to leakage in the slice direction, hence improving image quality.

FIG. 4

EP 1 580 575 A2

**Description**

The present invention relates to a cross-talk correction method for scintillators two-dimensionally arranged in a rectangular array, and an X-ray CT apparatus using the method.

In recent years, scintillators made of an inorganic crystal are used in an X-ray detector portion in an X-ray CT apparatus. The scintillators are disposed on a plane facing an X-ray tube that emits a cone-shaped X-ray beam spreading like a fan with a certain thickness, and they constitute an MD (multi-detector-row) CT apparatus. The MD CT apparatus acquires a three-dimensional (3D) image that has resolution in a direction of depth of a subject.

The scintillators emit fluorescent light of intensity proportional to that of entering X-rays, and the fluorescent light is converted into an electrical amount such as electric charge amount or current by photoelectric converters. At that time, the fluorescent light leaks between adjacent ones of the scintillators in a planar arrangement. Therefore, the electrical amount output from the photoelectric converters contains the leaked light.

The leakage occurs between adjacent ones of the scintillators arranged in a rectangular plane, and the effect of the leakage on an acquired tomographic image is different in the MD CT apparatus between a slice direction that corresponds to a direction of thickness of the X-ray beam, and a channel direction that corresponds to a direction of the spread of the fan. The slice direction for the scintillators in a planar arrangement generally coincides with a direction of depth of a bore in which the subject is situated.

The leakage in the channel direction implies that information leaks between projection information based on which a tomographic image is reconstructed, chiefly resulting in reduction in spatial resolution of the tomographic image. So several kinds of hardware and image processing techniques for improving spatial resolution also provide an effect of reducing the leakage of fluorescent light in the channel direction (for example, see Japanese Patent Application Laid Open No. S53-067394 (pp. 1 — 4, Figures 1 — 6)).

According to such background technology, however, image degradation due to leakage between scintillators adjacent in the slice direction cannot be prevented. Specifically, while the leakage in the slice direction appears as artifacts in a tomographic image, the phenomenon appearing on the tomographic image is different from that of leakage in the channel direction, and therefore, a similar image processing technique cannot be used.

Especially when a portion exhibiting a steep variation in the projection length in a subject, such as the neck or chest of the subject, is included in the slice direction in a range imaged by an MD CT apparatus, artifacts resulting from the leakage in the slice direction appear in the central portion of a tomographic image, thus hampering interpretation of the tomographic image.

For these reasons, it is important to somehow implement a cross-talk correction method and an X-ray CT apparatus capable of easily removing leakage of fluorescent light occurring between scintillators adjacent in the slice direction.

Therefore, an object of the present invention is to provide a cross-talk correction method and an X-ray CT apparatus capable of easily removing leakage of fluorescent light occurring between scintillators adjacent in the slice direction.

To solve the aforementioned problem and attain the purpose, a cross-talk correction method in accordance with the invention of a first aspect is characterized in comprising: when a plurality of scintillators for detecting intensity of an X-ray beam spreading like a fan with a certain thickness are present as a two-dimensional array arranged in a rectangular plane generally orthogonal to a direction of impingement of said X-ray beam, and information on said X-ray beam detected by each said scintillator contains both intensity information that is proportional to the intensity of the X-ray beam striking said scintillator and leakage information from a scintillator adjacent in a slice direction of said two-dimensional array that is a direction of said thickness, which leakage information is proportional to the intensity of the X-ray beam striking said adjacent scintillator, a step of calculating leakage coefficients for use in evaluating the amount of said leakage information from the intensity of the X-ray beam striking said adjacent scintillator, separately for a first leakage coefficient in a first direction along said slice direction and a second leakage coefficient in a second direction opposite to said first direction; and a step of removing said leakage information contained in the information detected at said plurality of scintillators using said leakage coefficients to determine said intensity information.

According to the invention of the first aspect, leakage coefficients for use in evaluating the amount of leakage information are calculated from the intensity of an X-ray beam striking an adjacent scintillator, separately for a first leakage coefficient in a first direction along the slice direction and a second leakage coefficient in a second direction opposite to the first direction, and the leakage coefficients are used to remove leakage information contained in information detected at the plurality of scintillators to determine intensity information.

A cross-talk correction method in accordance with the invention of a second aspect is characterized in that: said step of calculating comprises using Equation (1):

$$-\ln(D_n) \approx -\ln(S_n) + \varepsilon_+ \cdot (S_{n-1} / S_n - 1) + \varepsilon_- \cdot (S_{n+1} / S_n - 1),$$

where said first leakage coefficient is designated as $\varepsilon_+$, said second leakage coefficient as $\varepsilon_-$, a serial index of a scintillator in said slice direction as $n$, intensity information for said $n$-th scintillator as $D_n$, and detected information for said $n$-th scintillator as $S_n$.

According to the invention of the second aspect, the calculation is carried out using Equation (1).

A cross-talk correction method in accordance with the invention of a third aspect is characterized in that: said step of calculating comprises using slope phantom projection information that is detected information $S_n$ on an X-ray beam passing through a cylindrical phantom, said phantom having a circular diameter varying corresponding to the position in said slice direction.

According to the invention of the third aspect, the calculation is carried out using slope phantom projection information.

A cross-talk correction method in accordance with the invention of a fourth aspect is characterized in that: said step of calculating comprises conducting first function fitting on values corresponding to positions of said two-dimensional array in the channel direction that is a direction of the spread of said fan, at said n-th position in the slice direction of said slope phantom projection information, and using a function value obtained by said first function fitting as a value of $D_n$ in said Equation (1).

According to the invention of the fourth aspect, a function value obtained by first function fitting effected in the channel direction on the slope phantom projection information is used as a value of $D_n$ in Equation (1) for the calculation.

A cross-talk correction method in accordance with the invention of a fifth aspect is characterized in that: said function is a quadratic function.

According to the invention of the fifth aspect, the function removes a cross-talk component.

A cross-talk correction method in accordance with the invention of a sixth aspect is characterized in that: said step of calculating comprises conducting second function fitting applying said Equation (1) to said slope phantom projection information and said function value at the same position in the channel direction, and determining said first leakage coefficient $\varepsilon_+$ and said second leakage coefficient $\varepsilon_-$ from a function form obtained by said second function fitting.

According to the invention of the sixth aspect, the calculation determines first leakage coefficient $\varepsilon_+$ and second leakage coefficient $\varepsilon_-$ from a function form obtained by the second function fitting in which the slope phantom projection information and the function value obtained by the first function fitting is applied to Equation (1).

A cross-talk correction method in accordance with the invention of a seventh aspect is characterized in that: said function fitting is achieved using a method of least squares or regression analysis.

According to the invention of the seventh aspect, optimization is obtained by the function fitting.

A cross-talk correction method in accordance with the invention of an eighth aspect is characterized in that: said step of removing comprises determining said intensity information $D_n$ using Equation (2):

$$D_n = (S_n - \varepsilon_+ \cdot S_{n-1} - \varepsilon_- \cdot S_{n+1}) / g$$

where said first leakage coefficient is designated as $\varepsilon_+$, said second leakage coefficient as $\varepsilon_-$, a serial index of a scintillator in said slice direction as $n$, detected information for said $n$-th scintillator as $S_n$, intensity information for said $n$-th scintillator as $D_n$, and $g = (\varepsilon_+ + \varepsilon_-)$.

According to the invention of the eighth aspect, the removal is achieved using Equation (2).

A cross-talk correction method in accordance with the invention of a ninth aspect is characterized in that: said step of removing is conducted before or after sensitivity correction on said scintillators.

According to the invention of the ninth aspect, the removal is conducted before or after correction on the detector in the channel direction.

A cross-talk correction method in accordance with the invention of a tenth aspect is characterized in that: when said two-dimensional array of a plurality of scintillators is composed of a combination of a plurality of tiles, each tile being comprised of scintillators two-dimensionally arranged in a rectangular array, said step of calculating comprises determining first and second leakage coefficients for a single tile or for a plurality of said tiles.

According to the invention of the tenth aspect, the calculation is conducted for a single tile or for a plurality of tiles.

A cross-talk correction method in accordance with the invention of an eleventh aspect is characterized in that: said step of removing comprises determining said intensity information using first and second leakage coefficients for a single tile or for a plurality of said tiles.

According to the invention of the eleventh aspect, the removal is conducted for a single tile or for a plurality of tiles.

An X-ray CT apparatus in accordance with the invention of a twelfth aspect comprises an X-ray tube for emitting a cone-shaped X-ray beam spreading like a fan with a certain thickness, scintillators arranged as a two-dimensional array in a plane generally orthogonal to a direction of emission of said X-ray beam, for detecting said X-ray beam, and a data processing apparatus for reconstructing a tomographic image of a subject situated between said X-ray tube and said scintillators, based on two-dimensional projection information detected at said scintillators, and said X-ray CT apparatus is characterized in that said data processing apparatus comprises: calculating means for, when information detected by each said scintillator contains both intensity information that is proportional to the intensity of the X-ray beam striking said scintillator and leakage information from a scintillator adjacent in a slice direction of said two-dimensional array that is a direction of said thickness, which leakage information is proportional to the intensity of the X-ray beam striking said adjacent scintillator, calculating leakage coefficients for use in evaluating the amount of said leakage information from the intensity of the X-ray beam striking said adjacent scintillator, separately for a first leakage coefficient in a first direction along said slice direction and a second leakage coefficient in a second direction opposite to said first direction; and correcting means for removing said leakage information contained in said detected projection information using said leakage coefficients to determine said intensity information.

According to the invention of the twelfth aspect, when information detected by a scintillator contains both intensity information that is proportional to the intensity of the X-ray beam striking the scintillator and leakage information from a scintillator adjacent in a slice direction of a two-dimensional array that is a direction of thickness of the X-ray beam, which leakage information is proportional to the intensity of the X-ray beam striking the adjacent scintillator, in the data processing apparatus, the calculating means calculates leakage coefficients for use in evaluating the amount of leakage information from the intensity of the X-ray beam striking the adjacent scintillator, separately for a first leakage coefficient in a first direction along the slice direction and a second leakage coefficient in a second direction opposite to the first direction, and using the leakage coefficients, the correcting means removes the leakage information contained in the detected projection information to determine the intensity information.

An X-ray CT apparatus in accordance with the invention of a thirteenth aspect is characterized in that: said calculating means uses Equation (1):

$$-\ln(D_n) \approx -\ln(S_n) + \varepsilon_+ \cdot (S_{n-1} / S_n - 1) + \varepsilon_- \cdot (S_{n+1} / S_n - 1),$$

where said first leakage coefficient is designated as $\varepsilon_+$, said second leakage coefficient as $\varepsilon_-$, a serial index of a scintillator in said slice direction as $n$, intensity information for said n-th scintillator as $D_n$, and detected information for said n-th scintillator as $S_n$.

According to the invention of the thirteenth aspect, the calculating means uses Equation (1).

An X-ray CT apparatus in accordance with the invention of a fourteenth aspect is characterized in that: said calculating means uses slope phantom projection information that is detected information $S_n$ on an X-ray beam passing through a cylindrical phantom, said phantom having a circular diameter varying corresponding to the position in said slice direction.

According to the invention of the fourteenth aspect, the calculating means uses slope phantom projection information.

An X-ray CT apparatus in accordance with the invention of a fifteenth aspect is characterized in that: said calculating means comprises first function fitting means for conducting function fitting on values corresponding to positions of said two-dimensional array in the channel direction that is a direction of the spread of said fan, at said n-th position in the slice direction of said slope phantom projection information, and determining a function value obtained by said fitting as a value of $D_n$ in said Equation (1).

According to the invention of the fifteenth aspect, in the calculating means, the first function fitting means uses a function value obtained by function fitting conducted in the channel direction on the slope phantom projection information as a value of $D_n$ in Equation (1) for the calculation.

An X-ray CT apparatus in accordance with the invention of a sixteenth aspect is characterized in that: said calculating means comprises second function fitting means for conducting fitting applying said Equation (1) to said slope phantom projection information and said function value at the same position in the channel direction, and determining said first leakage coefficient $\varepsilon_+$ and said second leakage coefficient $\varepsilon_-$ from a function form of Equation (1) obtained by said fitting.

According to the invention of the sixteenth aspect, in the calculation means, the second function fitting means determines first leakage coefficient $\varepsilon_+$ and second leakage coefficient $\varepsilon_-$ from a function form obtained by fitting the slope phantom projection information and the function value obtained by the first function fitting to Equation (1).

An X-ray CT apparatus in accordance with the invention of a seventeenth aspect is characterized in that: said removing means determines said intensity information $D_n$ using Equation (2):

$$D_n = (S_n - \varepsilon_+ \cdot S_{n-1} - \varepsilon_- \cdot S_{n+1}) / g$$

where said first leakage coefficient is designated as $\varepsilon_+$, said second leakage coefficient as $\varepsilon_-$, a serial index of a scintillator in said slice direction as *n*, detected information for said *n*-th scintillator as $S_n$, intensity information for said n-th scintillator as $D_n$, and $g = (\varepsilon_+ + \varepsilon_-)$.

According to the invention of the seventeenth aspect, the removing means uses Equation (2).

An X-ray CT apparatus in accordance with the invention of an eighteenth aspect is characterized in that: said removing means works before or after sensitivity correction on said scintillators.

According to the invention of the eighteenth aspect, said removing means works before or after sensitivity correction on the detector.

An X-ray CT apparatus in accordance with the invention of a nineteenth aspect is characterized in that: when said two-dimensional array of a plurality of scintillators is composed of a combination of a plurality of tiles, each tile being comprised of scintillators two-dimensionally arranged in a rectangular array, said calculating means determines first and second leakage coefficients for a single tile or for a plurality of said tiles.

According to the invention of the nineteenth aspect, the calculating means conducts the calculation for a single tile or for a plurality of tiles.

An X-ray CT apparatus in accordance with the invention of a twentieth aspect is characterized in that: said removing means determines said intensity information using first and second leakage coefficients for a single tile or for a plurality of said tiles.

According to the invention of the twentieth aspect, the removing means conducts the removal for a single tile or for a plurality of tiles.

According to the present invention as described above, when information detected by a scintillator contains both intensity information that is proportional to the intensity of the X-ray beam spreading like a fan with a certain thickness striking the scintillator and leakage information from a scintillator adjacent in a slice direction of a two-dimensional array that is a direction of the thickness, which leakage information is proportional to the intensity of the X-ray beam striking the adjacent scintillator, in the data processing apparatus, the calculating means calculates leakage coefficients for use in evaluating the amount of leakage information from the intensity of the X-ray beam striking the adjacent scintillator, separately for a first leakage coefficient in a first direction along the slice direction and a second leakage coefficient in a second direction opposite to the first direction, and using the leakage coefficients, the correcting means removes the leakage information contained in the detected projection information to determine the intensity information; thus, information on leakage of fluorescent light occurring between scintillators adjacent in the slice direction is easily removed to obtain only intensity information that is proportional to the intensity of incoming X-rays and eliminate artifacts appearing in a tomographic image, hence improving image quality.

Further objects and advantages of the present invention will be apparent from the following description of the preferred embodiments of the invention as illustrated in the accompanying drawings, in which:

Figure 1 is a block diagram showing the overall configuration of an X-ray CT apparatus.

Figure 2 is a diagram showing an X-ray tube and an X-ray detector in accordance with an embodiment of the invention.

Figure 3 is a diagram showing leakage between solid-state detectors adjacent in the slice direction in accordance with the embodiment.

Figure 4 is a functional block diagram showing a data processing apparatus in accordance with the embodiment.

Figure 5 shows a slope phantom in accordance with the embodiment.

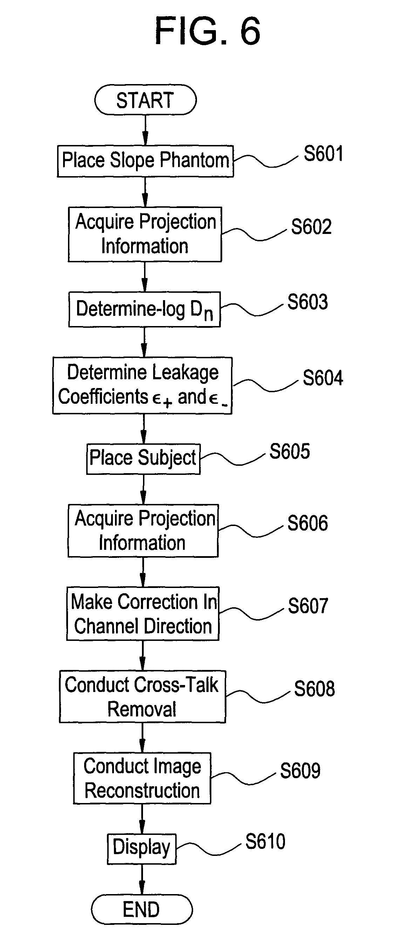

Figure 6 shows the operation of the X-ray CT apparatus in accordance with the embodiment.

Figure 7 shows an example of an X-ray detector comprised of a plurality of tiles.

The best mode for implementing a cross-talk correction method and an X-ray CT apparatus in accordance with the present invention will now be described with reference to the accompanying drawings. It should be noted that the present invention is not limited to the embodiment.

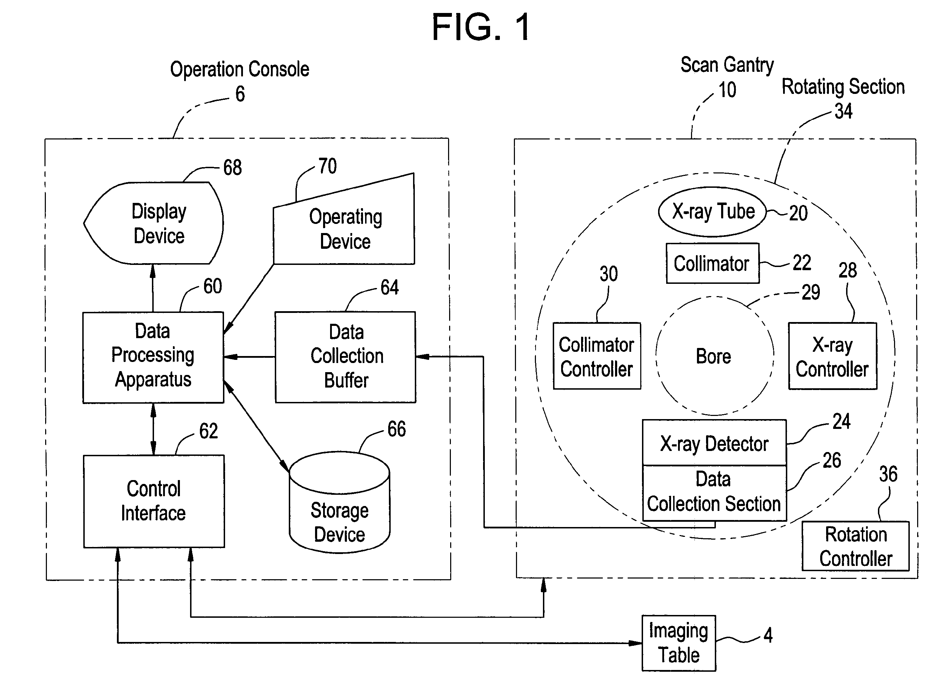

The overall configuration of an X-ray CT apparatus in accordance with an embodiment will first be described. Figure 1 shows a block diagram of an X-ray CT apparatus. As shown in Figure 1, the present apparatus comprises a scan gantry 10 and an operation console 6.

The scan gantry 10 has an X-ray tube 20. X-rays (not shown) emitted from the X-ray tube 20 are shaped by a collimator 22 into, for example, a cone-shaped X-ray beam that spreads like a fan with a certain thickness, and the beam is cast upon an X-ray detector 24.

The X-ray detector 24 has a plurality of scintillators arranged in a matrix extending in a width direction of the fan-beam X-rays. The X-ray detector 24 is configured as a multi-channel detector having a certain width, in which a plurality of scintillators are arranged in a matrix.

The X-ray detector 24 generally forms a concaved X-ray reception surface. The X-ray detector 24 is made of, for example, a combination of scintillators formed of an inorganic crystal and photodiodes serving as photoelectric converters.

The X-ray detector 24 is connected with a data collecting section 26. The data collecting section 26 collects information detected by individual scintillators in the X-ray detector 24. Emission of X-rays from the X-ray tube 20 is controlled by an X-ray controller 28. The interconnection between the X-ray tube 20 and X-ray controller 28, and that between the collimator 22 and a collimator controller 30 are omitted in the drawing. The collimator 22 is controlled by the collimator controller 30.

The X-ray tube 20, collimator 22, X-ray detector 24, data collecting section 26, X-ray controller 28 and collimator controller 30 are mounted on a rotating section 34 of the scan gantry 10. A subject or phantom is placed on an imaging table 4 in a bore 29 in the center of the rotating section 34. The rotating section 34 rotates under control by a rotation controller 36, emits X-rays at the X-ray tube 21, and detects X-rays passing through the subject or phantom at the X-ray detector 24 as projection information for each view corresponding to a rotation angle. The interconnection between the rotating section 34 and rotation controller 36 is omitted in the drawing.

The operation console 6 has a data processing apparatus 60. The data processing apparatus 60 comprises, for example, a computer. The data processing apparatus 60 is connected with a control interface 62. The control interface 62 is connected with the scan gantry 10. The data processing apparatus 60 controls the scan gantry 10 via the control interface 62.

The data collecting section 26, X-ray controller 28, collimator controller 30 and rotation controller 36 in the scan gantry 10 are controlled via the control interface 62. The individual interconnections between these sections and the control interface 62 are omitted in the drawing.

The data processing apparatus 60 is also connected with a data collection buffer 64. The data collection buffer 64 is connected with the data collecting section 26 in the scan gantry 10. Data collected by the data collecting section 26 are input to the data processing apparatus 60 via the data collection buffer 64.

The data processing apparatus 60 performs image reconstruction using transmitted X-ray signals, i.e., projection information, collected via the data collection buffer 64. The data processing apparatus 60 is also connected with a storage device 66. The storage device 66 stores projection information collected in the data collection buffer 64, information on reconstructed tomographic images, and programs for implementing the functions of the present apparatus.

The data processing apparatus 60 is further connected with a display device 68 and an operating device 70. The display device 68 displays tomographic image information output from the data processing apparatus 60 and other information. The operating device 70 is operated by a human operator to supply several kinds of instructions and information to the data processing apparatus 60. The operator uses the display device 68 and operating device 70 to interactively operate the present apparatus. The scan gantry 10, imaging table 4 and operation console 6 acquire a tomographic image by imaging a subject or phantom.

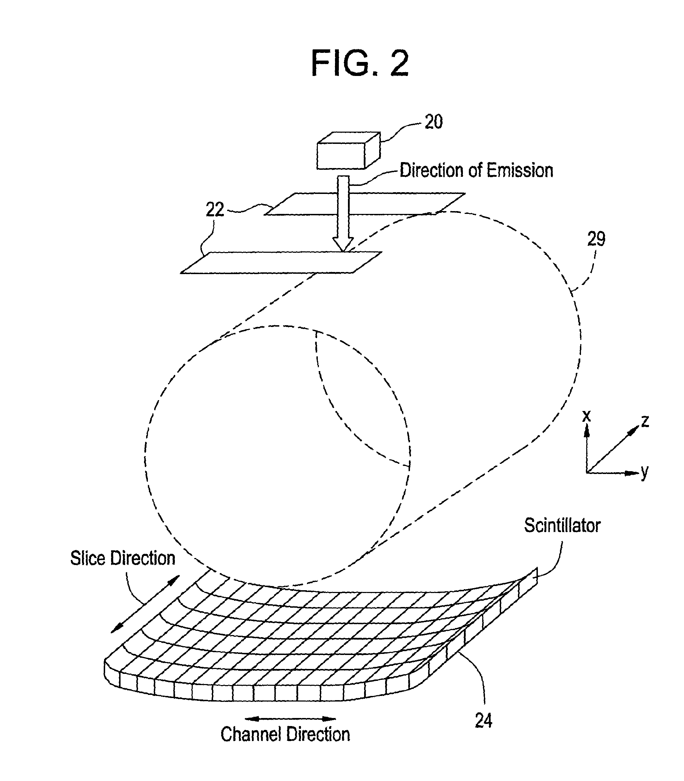

Figure 2 is a schematic diagram showing the spatial conformation of the X-ray tube 20, collimator 22 and X-ray detector 24. The X-ray detector 24 is comprised of scintillators arranged in a two-dimensional rectangular array on a surface facing a cone-shaped X-ray beam spreading like a fan with a certain thickness generated by the X-ray tube 20. The two-dimensional array has a slice direction that corresponds to the direction of thickness of the X-ray beam, and a channel direction that corresponds to the direction of width of the fan. The slice direction generally coincides the direction of depth through which the bore 29 runs. The X-ray detector 24 also forms a concave surface in the channel direction so that the incoming X-ray beam impinges orthogonally to the surface of the two-dimensional array. The X-ray detector 24 is comprised of, for example, 64 rows in the slice direction and 1000 channels in the channel direction, of scintillators. The back surfaces of the scintillators are provided with the same number of photoelectric converters (not shown).

Now description will be made on information detected at a photoelectric converter when an X-ray beam strikes a scintillator, and a cross-talk correction method in accordance with the present invention. Upon a strike by X-rays, a scintillator emits fluorescent light of intensity proportional to that of the X-rays. On the other hand, since the X-ray detector 24 has scintillators densely arranged in a two-dimensional array, leakage of fluorescent light inevitably occurs between adjacent scintillators.

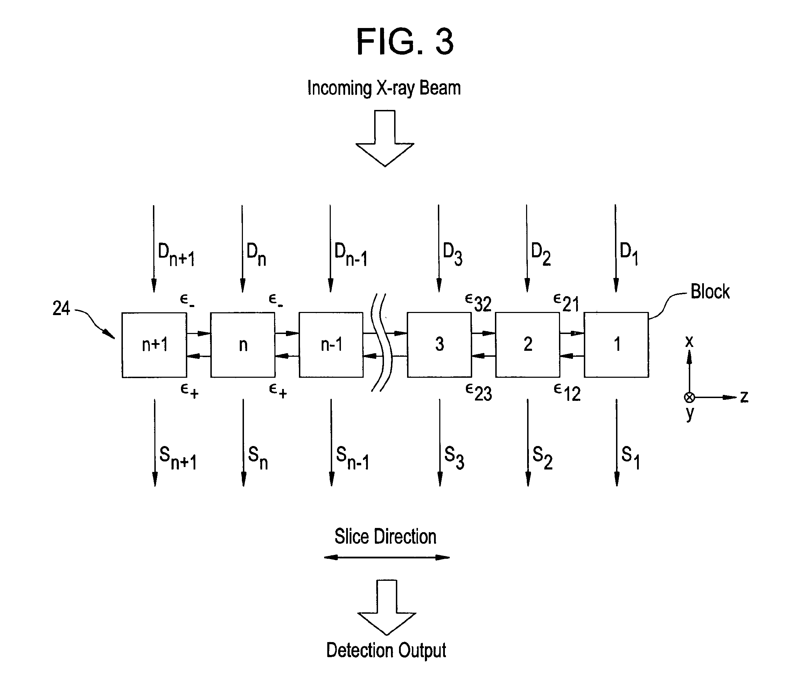

Figure 3 is a diagram showing a model of leakage of fluorescent light occurring between scintillators adjacent in the slice direction. The scintillators and photoelectric converters in the X-ray detector 24 are represented by blocks serially numbered starting from one at an end in the slice direction. An arbitrary serial number is designated as $n$. In an n-th block, an output proportional to the intensity of an incoming X-ray beam is defined as intensity information $D_n$ (which, in Figure 3, is shown as the intensity of the incoming X-ray beam), and an output observed as a result at an individual block is designated as detected information $S_n$. It should be noted that the intensity information $D_n$ and detected information $S_n$ have a subscript $n$ indicating the serial number corresponding to the block number.

The leakage of fluorescent light between adjacent scintillators in this model will now be described with reference to Figure 3. While the description will be made mainly on a second block numbered two, the same applies to other blocks. First, leakage proportional to the intensity $D_2$ of an X-ray beam entering the block 2 occurs in adjacent blocks 1 and 3. The magnitude of the leakage is $\varepsilon 21 \cdot D_2$ into the block 1 and $\varepsilon 23 \cdot D_2$ into the block 3, where leakage coefficients for the leakage corresponding to the intensity $D_2$ into the blocks 1 and 3 are represented by $\varepsilon 21$ and $\varepsilon 23$, respectively.

Moreover, the block 2 also receives leaked light from the adjacent blocks 1 and 3. The leaked light from the block 1 into block 2 that is proportional to the intensity $D_1$ of an X-ray beam entering the block 1 is $\varepsilon 12 \cdot D_1$, where the leakage coefficient is represented by $\varepsilon 12$, and the leaked light from the block 3 into block 2 that is proportional to the intensity $D_3$ of an X-ray beam entering the block 3 is $\varepsilon 32 \cdot D_3$, where the leakage coefficient is represented by $\varepsilon 32$.

Therefore, detected information $S_2$ at the block 2 is given by:

$$S_2 = D_2 - \varepsilon_{21} \cdot D_2 - \varepsilon_{23} \cdot D_2 + \varepsilon_{12} \cdot D_1 + \varepsilon_{32} \cdot D_3$$

$$= (1 - \varepsilon_{21} - \varepsilon_{23}) \cdot D_2 + \varepsilon_{12} \cdot D_1 + \varepsilon_{32} \cdot D_3.$$

It is empirically known that owing to a cause associated with the manufacturing process of the X-ray detector 24, the leakage coefficient is different between orientations in the slice direction, and it varies not much from block to block. In other words,

$$\varepsilon_{12} = \varepsilon_{23} = \varepsilon_+,$$

and

$$\varepsilon_{21} = \varepsilon_{32} = \varepsilon_-$$

hold, where $\varepsilon_+$ is a first leakage coefficient and represents a leftward leakage coefficient in the slice direction in Figure 3, and $\varepsilon_-$ is a second leakage coefficient and represents a rightward leakage coefficient in the slice direction in Figure 3. Figure 3 shows the leakage coefficients indicated by symbols $\varepsilon_+$ and $\varepsilon_-$ in its left portion.

Thus, the detected information $S_2$ at the block 2 is given by:

$$S_2 = (1 - \varepsilon_+ - \varepsilon_-) \cdot D_2 + \varepsilon_+ \cdot D_1 + \varepsilon_- \cdot D_3.$$

Likewise, for detected information $S_n$ at an n-th block $n$, Equation (3) holds as follows:

$$S_n = (1 - \varepsilon_+ - \varepsilon_-) \cdot D_n + \varepsilon_+ \cdot D_{n-1} + \varepsilon_- \cdot D_{n+1}. \tag{3}$$

In Equation (3), the detected information $S_2$ is an amount experimentally detected as projection information, and the intensity information $D_n$ is an amount proportional to a true incoming X-ray intensity calculated based on a formula. Therefore, $n$ number of Equation (3)'s hold for $n$ blocks, and $n$ pieces of intensity information $D_n$ can be determined in principle from $n$ pieces of detected information $S_2$. However, since the right-hand side of Equation (3) includes a plurality of pieces of intensity information $D_n$ as unknowns, they are difficult to evaluate.

Therefore, Equation (3) is simplified into a function form that facilitates determination of intensity information $D_n$ from detected information $S_2$ as follows. If it is assumed that $D_n = (D_{n-1}+D_{n+1})/2$, Equation (3) can be transformed into an expression as follows:

$$S_n = D_n + (\varepsilon_+ - \varepsilon_-) \cdot (D_{n-1} - D_{n+1}) / 2.$$

In this equation, the leakage coefficients $\varepsilon_+$ and $\varepsilon_-$ are experimentally evaluated as a small value of the order of 0.1. Moreover, since their difference is very small, the second term on the right-hand side of the equation above may be neglected, thus resulting in:

$$S_n \approx D_n. \tag{4}$$

Substituting Equation (4) into Equation (3), we have:

$$S_n \approx (1 - \varepsilon_+ - \varepsilon_-) \cdot D_n + \varepsilon_+ \cdot S_{n-1} + \varepsilon_- \cdot S_{n+1}.$$

The equation is solved for $D_n$ to give Equation (2) as follows:

$$D_n = (S_n - \varepsilon_+ \cdot S_{n-1} - \varepsilon_- \cdot S_{n+1}) / g, \tag{2}$$

where $g = 1 - \varepsilon_+ - \varepsilon_-$.

Equation (2) contains on its right-hand side detected information $S_{n+1}$, $S_n$ and $S_{n-1}$ only that are experimentally detected as projection information, and allows the intensity information $D_n$ to be easily calculated if only the leakage coefficients $\varepsilon_+$ and $\varepsilon_-$ are determined. Thus, second function fitting means, which will be discussed later, uses Equation (2) to calculate X-ray beam intensity information $D_n$ for each block, from the leakage coefficients $\varepsilon_+$ and $\varepsilon_-$ calculated using detected information $S_n$ that is actually observed projection information and a method that will be discussed below.

Now a method of determining leakage coefficients $\varepsilon_+$ and $\varepsilon_-$ for use in determining intensity information $D_n$ for each block will be described. When leakage coefficients $\varepsilon_+$ and $\varepsilon_-$ are unknown, Equation (2) cannot be solved because intensity information $D_n$ is unknown.

Thus, a logarithm for both sides of Equation (2) is taken, and approximation is effected by using an approximation formula for a logarithm function $\ln(1 + x) \approx x$, to give Equation (1) as follows:

$$-\ln(D_n) \approx -\ln(S_n) + \varepsilon_+ \cdot (S_{n-1} / S_n - 1) + \varepsilon_- \cdot (S_{n+1} / S_n - 1). \tag{1}$$

The $-\ln(D_n)$ term on the left-hand side of Equation (1) represents the projection length of the X-ray beam entering each block. The term $-\ln(D_n)$ denotes the projection length in an ideal case when no leakage occurs in the slice direction. On the other hand, $-\ln(S_n)$, which is the actually observed projection length, contains leakage in the slice direction. If any leakage is present in the slice direction, it is known from experience that partial degradation of projection information occurs concentratedly in a central portion of projection information in the channel direction. Thus, the partial degradation due to leakage in the slice direction is removed by first function fitting, which will be discussed later, on projection information in the channel direction, to extract ideal projection information only. The fitted function value is then used as $-\ln(D_n)$ for an ideal case in which no leakage occurs in the slice direction as described above. Equation (1) then becomes an equation including the leakage coefficients $\varepsilon_+$ and $\varepsilon_-$ only as unknowns.

Moreover, when Equation (1) is used to determine the leakage coefficients $\varepsilon_+$ and $\varepsilon_-$, there arises a requirement that $S_{n-1}/S_n \neq 1$ and $S_{n+1}/S_n \neq 1$ on the right-hand side. This requirement is satisfied by using projection information of a slope phantom, which will be discussed later, as detected information $S_n$.

For Equation (1), a number, equal to the number of blocks for detected information $S_n$, of the equations stand for two unknowns, i.e., leakage coefficients $\varepsilon_+$ and $\varepsilon_-$. Therefore, a method of least squares or regression analysis including the method of least squares can be used to determine optimum values for the leakage coefficients $\varepsilon_+$ and $\varepsilon_-$ from the plurality of equations or a large number of pieces of detected information $S_n$.

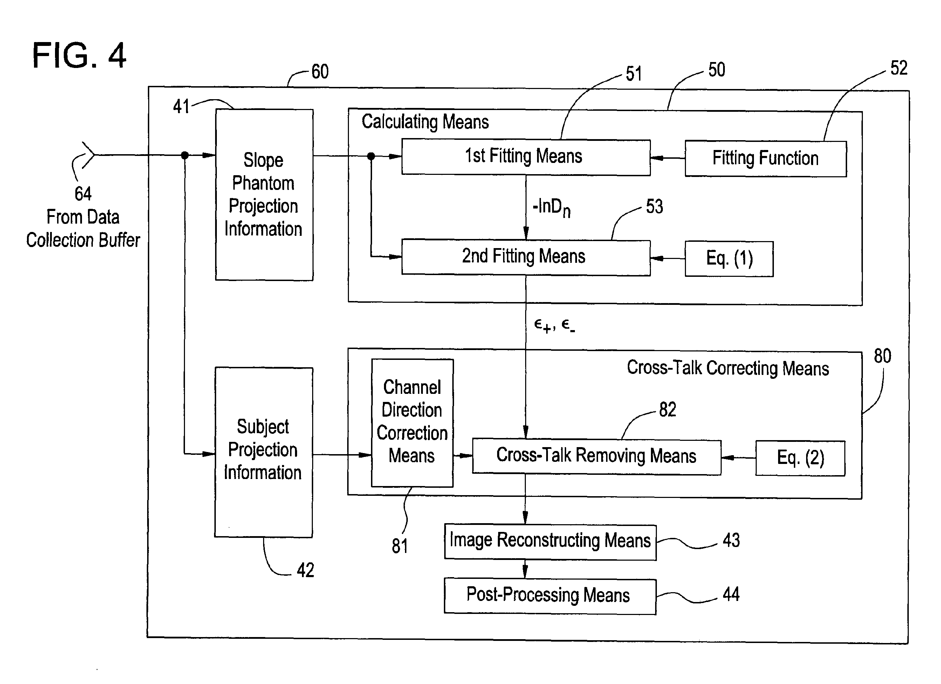

Figure 4 is a functional block diagram showing the data processing apparatus 60 for implementing the above-described cross-talk correction method. The data processing apparatus 60 comprises slope phantom projection information 41, calculating means 50, subject projection information 42, cross-talk correcting means 80, image reconstructing means 43, and post-processing means 44. The calculating means 50 comprises first function fitting means 51, a fitting function 52, second function fitting means 53, and Equation (1); and the cross-talk correcting means 80 comprises channel direction correction means 81, cross-talk removing means 82, and Equation (2).

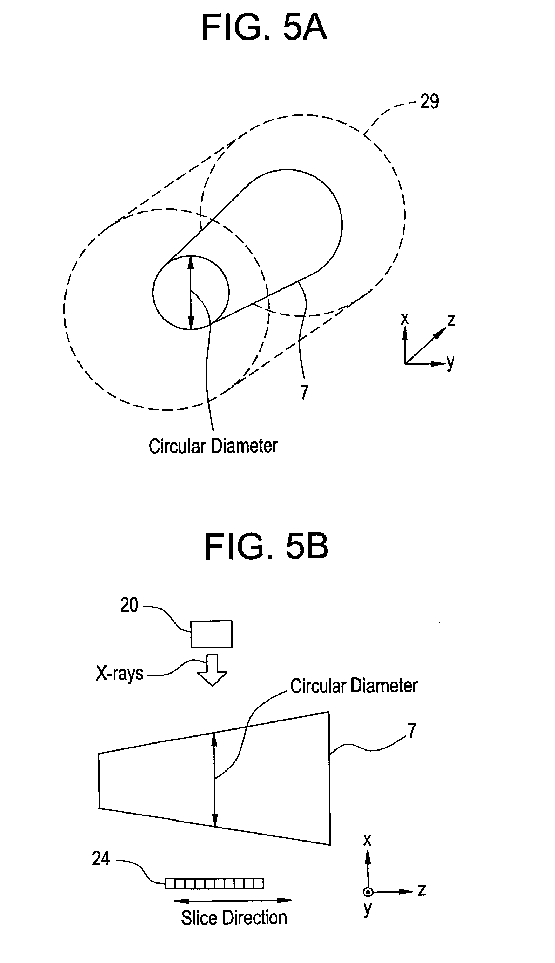

The slope phantom projection information 41 is projection information input from the data collection buffer 64, acquired when a slope phantom is situated in the central portion of the bore 29. Figure 5 exemplarily shows a slope phantom 7. Figure 5(A) shows the slope phantom 7 disposed in the bore 29. The slope phantom 7 has a cylindrical shape containing therein an X-ray absorptive material such as water, and the circular diameter of the cylinder varies in proportion to the position in the slice direction. Figure 5(B) shows a cross section of the slope phantom 7 in the slice direction. The circular diameter of the slope phantom 7 varies in proportion to the position in the slice direction. Thus, the scintillators in the X-ray detector 24 acquire projection information that differs from scintillator to scintillator, which information sequentially increases or decreases in the slice direction. The detected information $S_n$, in which $S_{n-1}/S_n = S_{n+1}/S_n \neq 1$, in the slice direction thus satisfies the requirement for deriving Equation (1). It should be understood that the slope phantom projection information 41 has a matrix data structure corresponding to the X-ray detector 24, with two indices, i.e., channel index and slice index.

Referring again to Figure 4, the calculating means 50 uses detected information $S_n$ making up the slope phantom projection information 41 to determine leakage coefficients $\varepsilon_+$ and $\varepsilon_-$ for the X-ray detector 24. The calculating means 50 drives the first function fitting means 51 to fit the data in the channel direction to the fitting function 52 for each position in the slice direction of the slope phantom projection information 41 to determine a function value for each channel. The second function fitting means 53 also fits Equation (1) to $-\ln(D_n)$, which is the fitted function value obtained at the first function fitting means 51, and data in the slice direction of the slope phantom projection information 41. The leakage coefficients $\varepsilon_+$ and $\varepsilon_-$ are thus obtained from the fitted function form of Equation (1). The first function fitting means 51 and second function fitting means 53 use fitting means such as a method of least squares or regression analysis incorporating the method of least squares to determine an optimum fitting function and coefficients for the function. For the fitting function 52, a quadratic function is employed, for example.

The subject projection information 42 is projection information input from the data collection buffer 64, acquired when a subject is situated in the central portion of the bore 29. As in the slope phantom projection information 41, the subject projection information 42 has a matrix data structure corresponding to the X-ray detector 24, with two indices, i.e., channel index and slice index.

The cross-talk correcting means 80 determines intensity information $D_n$ proportional to the X-ray beam intensity striking a scintillator from the detected information $S_n$ making up the subject projection information 42. The cross-talk correcting means 80 makes several kinds of correction on the subject projection information 42 by the channel direction correction means 81. The channel direction correction means 81 includes offset correction, logarithm transformation, X-ray dose correction and sensitivity correction (also known as detector sensitivity correction), and primarily removes error factors of the X-ray detector 24 in the channel direction.

The cross-talk removing means 82 uses the detected information $S_n$ making up the subject projection information 42 after the correction in the channel direction to determine intensity information $D_n$ in which cross-talk in the subject projection information 42 in the slice direction has been removed, from the leakage coefficients $\varepsilon_+$ and $\varepsilon_-$ determined at the calculating means 50 and Equation (2). The calculation is carried out by addition/subtraction/multiplication/division, using Equation (2).

The cross-talk removing means 82 may be positioned either before or after the sensitivity correction made by the channel direction correction means 81. The reason for this is as follows. In the sensitivity correction, correction on detector sensitivity is primarily done based on projection information acquired in the absence of the slope phantom 7 shown in Figure 5, i.e., acquired for only the air. Designating the detected information comprising the only-air projection information as An, variability in $A_n$ represents variability in sensitivity of the individual detectors, and the variability in sensitivity of the individual detectors is corrected by $S_n/A_n$ in the detected information $S_n$ of the subject.

Moreover, designating the detected information $A_n$ in which cross-talk in the slice direction has been removed as $I_n$,

$$I_n = (A_n - \varepsilon_+ \cdot A_{n-1} - \varepsilon_- \cdot A_{n+1}) / g$$

is obtained from Equation (2); however, considering that the projection information is on the air, $I_{n+1} = I_n = I_{n-1}$ and $A_{n+1} \approx A_n \approx A_{n-1}$, and hence,

$$I_n = A_n. \tag{4}$$

Equation (4) implies that there is no need to remove cross-talk in the slice direction from the detected information $A_n$ comprising projection information on only the air.

If cross-talk removal on the subject projection information 42 in the slice direction is conducted before sensitivity correction, from Equation (2), detected information An for use in the sensitivity correction is employed to determine detected information $I_n$ after cross-talk removal in the slice direction, and since

$$I_n = A_n,$$

from Equation (4), detected information $S_n$ on the subject is:

$$S_n = (S_n - \varepsilon_+ \cdot S_{n-1} - \varepsilon_- \cdot S_{n+1})/ g,$$

resulting in:

$$D_n = S_n / I_n = (S_n - \varepsilon_+ \cdot S_{n-1} - \varepsilon_- \cdot S_{n+1})/(g \cdot A_n) \tag{5}$$

from sensitivity-corrected intensity output $D_n = S_n / I_n$.

On the other hand, if cross-talk removal on the subject projection information 42 in the slice direction is conducted after sensitivity correction, Equation (2) is employed for detected information $S_n$ / An of the subject after sensitivity correction to remove cross-talk in the slice direction, which gives:

$$D_n = (S_n / A_n - \varepsilon_+ \cdot S_{n-1} / A_n - \varepsilon \cdot S_{n+1} / A_n)/ g$$

$$= (S_n - \varepsilon_+ \cdot S_{n-1} - \varepsilon_- \cdot S_{n+1})/(g \cdot A_n). \tag{6}$$

Equation (5) and Equation (6) are identical, and thus, the same result is obtained whether cross-talk removal on the subject projection information 42 in the slice direction is conducted before or after sensitivity correction.

The image reconstructing means 43 reconstructs a tomographic image of the subject using sinograms of intensity information $D_n$ at a plurality of views in which cross-talk in the slice direction has been corrected. In the image reconstruction, a filtered backprojection technique, for example, is employed.

The post-processing means 44 applies CT value conversion etc. on the reconstructed tomographic image information, and displays the reconstructed image on the display device 68.

Next, the operation of the X-ray CT apparatus in accordance with the present embodiment will be described. The operator first places the slope phantom 7 situated on the imaging table 4 in the central portion of the bore 29, as shown in Figure 5(A) (Step S601). The data processing apparatus 60 then controls the scan gantry 10 to acquire slope phantom projection information 41 on the slope phantom 7 (Step S602). The acquired slope phantom projection information 41 is transferred to the data collection buffer 64.

Thereafter, the data processing apparatus 60 acquires the slope phantom projection information 41 from the data collection buffer 64, and drives the calculating means 50 to determine -In($D_n$) of Equation (1) from the slope phantom projection information 41 (Step S603). At that time, the calculating means 50 determines a fitting function for each slice using the first function fitting means 51, and a logarithm of the fitting function value at each channel is defined as In($D_n$).

Thereafter, the data processing apparatus 60 drives the calculating means 50 to determine leakage coefficients $\varepsilon_+$ and $\varepsilon_-$ from the slope phantom projection information 41 and -In($D_n$) determined at the first function fitting means 51 (Step S604). At that time, the calculating means 50 determines optimum values for the unknown leakage coefficients $\varepsilon_+$ and $\varepsilon_-$ by the second function fitting means 53 applying detected information $S_n$ and -In($D_n$) at the same channel to Equation (1).

Thereafter, the operator places a subject situated on the imaging table 4 in the central portion of the bore 29 (Step S605). The data processing apparatus 60 then controls the scan gantry 10 to acquire subject projection information 42 for the subject (Step S606). The acquired subject projection information 42 is transferred to the data collection buffer 64.

The data processing apparatus 60 then acquires the subject projection information 42 from the data collection buffer 64, and drives the channel direction correction means 81 to make correction in the channel direction (Step S607). Error factors contained in the detected information $S_n$ of the subject projection information 42 in the channel direction is thus removed.

The cross-talk correcting means 80 then uses the cross-talk removing means 82 to conduct cross-talk removal in the slice direction from the subject projection information 42 after the correction in the channel direction (Step S608). Intensity information $D_n$ is thus obtained which is removed of a factor of leakage of fluorescent light contained in the detected information $S_n$ of the subject projection information 42 in the slice direction.

Thereafter, the image reconstructing means 43 uses the intensity information $D_n$ for each channel to conduct image reconstruction (Step S609). The post-processing means 44 then displays the reconstructed image information on the display device 68 (Step S610), and the processing is terminated.

As described above, in the present embodiment, the first function fitting means 51 in the channel direction and the second function fitting means 53 in the slice direction determine an ideal projection length, $\ln(D_n)$, from slope phantom projection information 41 on the slope phantom 7, Equation (1) is then used to determine the leakage coefficients $\varepsilon_+$ and $\varepsilon_-$, and subsequently, the cross-talk removing means 82 determines intensity information $D_n$ from detected information $S_n$ making up subject projection information 42 on the subject using Equation (2) and the leakage coefficients $\varepsilon_+$ and $\varepsilon_-$; thus, the leakage component for scintillators adjacent in the slice direction contained in the subject projection information 42 is easily removed to eliminate artifacts due to leakage in the slice direction, hence improving image quality.

Moreover, while in the present embodiment, the leakage coefficients $\varepsilon_+$ and $\varepsilon_-$ of the X-ray detector 24 are different depending upon the orientation in the slice direction, they may be differentiated at the same time between tiles making up the X-ray detector 24. The term "tile" refers to a matrix-like arrangement of scintillators, and a plurality of such tiles are combined to constitute one X-ray detector 24.

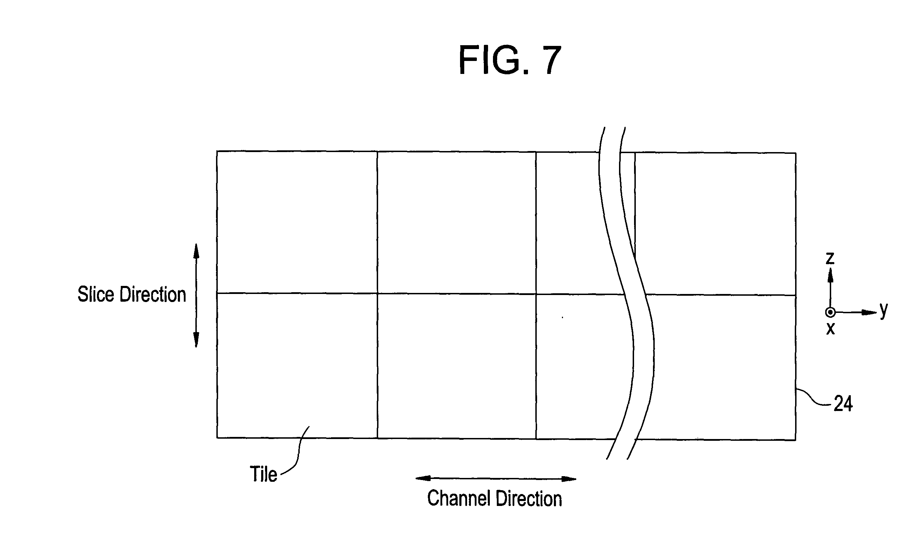

Figure 7 shows an example of the X-ray detector 24 in which two tiles are arranged in the slice direction and a certain plural number of tiles are arranged in the channel direction. The tiles have the same shape, and one tile is comprised of 32 rows in the slice direction and 16 columns in the channel direction, of scintillators. Since the scintillators are made by the same manufacturing process, the property of the scintillators is consistent within a tile and their leakage coefficients $\varepsilon_+$ and $\varepsilon_-$ have similar values from scintillator to scintillator. The cross-talk correction method described hereinabove is then applied to a single tile or to a plurality of tiles arranged in the same column in the slice direction to achieve correction with higher precision.

**Claims**

1. A cross-talk correction method comprising:

   when a plurality of scintillators (24) for detecting intensity of an X-ray beam spreading like a fan with a certain thickness are present as a two-dimensional array (24) arranged in a rectangular plane generally orthogonal to a direction of impingement of said X-ray beam, and information on said X-ray beam detected by each said scintillator (24) contains both intensity information that is proportional to the intensity of the X-ray beam striking said scintillator (24) and leakage information from a scintillator (24) adjacent in a slice direction of said two-dimensional array (24) that is a direction of said thickness, which leakage information is proportional to the intensity of the X-ray beam striking said adjacent scintillator (24),
   a step of calculating leakage coefficients for use in evaluating the amount of said leakage information from the intensity of the X-ray beam striking said adjacent scintillator (24), separately for a first leakage coefficient in a first direction along said slice direction and a second leakage coefficient in a second direction opposite to said first direction; and
   a step of removing said leakage information contained in the information detected at said plurality of scintillators (24) using said leakage coefficients to determine said intensity information.

2. An X-ray CT apparatus comprising:

   an X-ray tube (20) for emitting a cone-shaped X-ray beam spreading like a fan with a certain thickness, scintillators (24) arranged as a two-dimensional array (24) in a plane generally orthogonal to a direction of emission of said X-ray beam, for detecting said X-ray beam, and
   a data processing apparatus (60) for reconstructing a tomographic image of a subject situated between said X-ray tube (20) and said scintillators (24), based on two-dimensional projection information detected at said scintillators (24),

   said X-ray CT apparatus wherein said data processing apparatus (60) comprises:

   a calculating device (50) for, when information detected by each said scintillator (24) contains both intensity information that is proportional to the intensity of the X-ray beam striking said scintillator (24) and leakage information from a scintillator (24) adjacent in a slice direction of said two-dimensional array (24) that is a direction of said thickness, which leakage information is proportional to the intensity of the X-ray beam striking said adjacent scintillator (24), calculating leakage coefficients for use in evaluating the amount of said leakage information from the intensity of the X-ray beam striking said adjacent scintillator (24), separately for a first leakage coefficient in a first direction along said slice direction and a second leakage coefficient in a second direction opposite to said first direction; and

a correcting device (80) for removing said leakage information contained in said detected projection information using said leakage coefficients to determine said intensity information.

3. The X-ray CT apparatus of claim 2, wherein:

said calculating device (50) uses Equation (1):

$$-\ln(D_n) \approx -\ln(S_n) + \varepsilon_+ \cdot (S_{n-1} / S_n - 1) + \varepsilon_- \cdot (S_{n+1} / S_n - 1),$$

where said first leakage coefficient is designated as $\varepsilon_+$, said second leakage coefficient as $\varepsilon_-$, a serial index of a scintillator (24) in said slice direction as $n$, intensity information for said $n$-th scintillator (24) as $D_n$, and detected information for said $n$-th scintillator (24) as $S_n$.

4. The X-ray CT apparatus of claim 3, wherein:

said calculating device (50) uses slope phantom projection information that is detected information $S_n$ on an X-ray beam passing through a cylindrical phantom (7), said phantom having a circular diameter varying corresponding to the position in said slice direction.

5. The X-ray CT apparatus of claim 4, wherein:

said calculating device (50) comprises a first function fitting device (51) for conducting function fitting on values corresponding to positions of said two-dimensional array (24) in the channel direction that is a direction of the spread of said fan, at said n-th position in the slice direction of said slope phantom projection information, and determining a function value obtained by said fitting as a value of $D_n$ in said Equation (1).

6. The X-ray CT apparatus of claim 5, wherein:

said calculating device (50) comprises a second function fitting device (53) for conducting fitting applying said Equation (1) to said slope phantom projection information and said function value at the same position in the channel direction, and determining said first leakage coefficient $\varepsilon_+$ and said second leakage coefficient $\varepsilon_-$ from a function form of Equation (1) obtained by said fitting.

7. The X-ray CT apparatus of any one of claims 2 — 6, wherein:

said correcting device (80) determines said intensity information $D_n$ using Equation (2):

$$D_n = (S_n - \varepsilon_+ \cdot S_{n-1} - \varepsilon_- \cdot S_{n+1}) / g$$

where said first leakage coefficient is designated as $\varepsilon_+$, said second leakage coefficient as $\varepsilon_-$, a serial index of a scintillator (24) in said slice direction as $n$, detected information for said n-th scintillator (24) as $S_n$, intensity information for said n-th scintillator (24) as $D_n$, and $g = (\varepsilon_+ + \varepsilon_-)$.

8. The X-ray CT apparatus of claim 7, wherein:

said correcting device (80) works before or after sensitivity correction on said scintillators (24).

9. The X-ray CT apparatus of any one of claims 2 — 8, wherein:

when said two-dimensional array (24) of a plurality of scintillators (24) is composed of a combination of a plurality of tiles, each tile being comprised of scintillators (24) two-dimensionally arranged in a rectangular array (24), said calculating device (50) determines first and second leakage coefficients for a single tile or for a plurality of said tiles.

10. The X-ray CT apparatus of claim 9, wherein:

said correcting device (80) determines said intensity information using first and second leakage coefficients for a single tile or for a plurality of said tiles.

# FIG. 1

Operation Console
6

Scan Gantry
10

Rotating Section
34

Display Device 68

Operating Device 70

X-ray Tube 20

Collimator 22

28

Collimator Controller 30

Bore 29

X-ray Controller

Data Processing Apparatus 60

Data Collection Buffer 64

X-ray Detector 24

Data Collection Section 26

36

Control Interface 62

Storage Device 66

Rotation Controller

Imaging Table 4

EP 1 580 575 A2

# FIG. 2

20

Direction of Emission

22

29

x

z

y

Slice Direction

Scintillator

24

Channel Direction

# FIG. 3

Incoming X-ray Beam

Slice Direction

Detection Output

EP 1 580 575 A2

# FIG. 4

FIG. 4 block diagram — showing (60) enclosing:

- (64) From Data Collection Buffer
- (41) Slope Phantom Projection Information
- (42) Subject Projection Information
- (51) 1st Fitting Means / (50) Calculating Means / (52) Fitting Function
- $-\ln D_n$
- (53) 2nd Fitting Means / Eq. (1)
- $\epsilon_+, \epsilon_-$
- (80) Cross-Talk Correcting Means
- (81) Channel Direction Correction Means
- (82) Cross-Talk Removing Means / Eq. (2)
- (43) Image Reconstructing Means
- (44) Post-Processing Means

EP 1 580 575 A2

# FIG. 5A

29

Circular Diameter

7

x
z
y

# FIG. 5B

20

X-rays

Circular Diameter

7

24

Slice Direction

x
z
y

EP 1 580 575 A2

# FIG. 6

START

Place Slope Phantom — S601

Acquire Projection Information — S602

Determine-log $D_n$ — S603

Determine Leakage Coefficients $\epsilon_+$ and $\epsilon_-$ — S604

Place Subject — S605

Acquire Projection Information — S606

Make Correction In Channel Direction — S607

Conduct Cross-Talk Removal — S608

Conduct Image Reconstruction — S609

Display — S610

END

19

EP 1 580 575 A2

FIG. 7

Slice Direction

Channel Direction

Tile

24

20